# EUROPÄISCHE PATENTSCHRIFT

(11) **EP 0 896 973 B1**
(45) Veröffentlichungstag und Bekanntmachung des Hinweises auf die Patenterteilung: **20.04.2005**
(21) Anmeldenummer: 98114372.0
(22) Anmeldetag: 31.07.1998
(51) Int. Cl.: C08G 18/79, C08K 5/52

(54) **Verfahren zur Herstellung farbheller Uretdionpolyisocyanate**
Process for the preparation of clear Uretdionpolyisocyanates
Procédé pour la préparation de polyisocyanates clairs contenant des groupes d'urethdione

(30) Priorität: 13.08.1997 DE 19735043
(43) Veröffentlichungstag der Anmeldung: 17.02.1999
(73) Patentinhaber: Bayer MaterialScience AG, 51368 Leverkusen (DE)
(72) Erfinder: Laas, Hans-Josef, Dr., 50733 Köln (DE); Halpaap, Reinhard, Dr., 51519 Odenthal (DE); Meier-Westhues, Hans-Ulrich, Dr., 51379 Leverkusen (DE); Freudenberg, Ulrich, Dr., 50259 Pulheim (DE)

(56) Entgegenhaltungen:
- EP-A- 0 377 177
- EP-A- 0 425 710
- EP-A- 0 431 331
- EP-A- 0 569 804
- EP-A- 0 643 042
- EP-A- 0 735 027
- EP-A- 0 744 421

## Beschreibung

Die Erfindung betrifft ein Verfahren zur Herstellung farbheller Uretdionpolyisocyanate sowie deren Verwendung als Ausgangskomponente für Polyurethankunststoffe, insbesondere als Isocyanatkomponente zur Herstellung von Uretdionpulverlackvernetzem.

Die Herstellung aliphatischer oder cycloaliphatischer Polyisocyanate mit Uretdionstruktur durch katalytische Dimerisierung und gegebenenfalls Trimerisierung (Oberbegriff: Oligomerisierung) monomerer aliphatischer oder cycloaliphatischer Diisocyanate ist bekannt (z.B. J. prakt. Chem. 336 (1994) 185 - 200).

Die nach diesen bekannten Verfahren erhältlichen Uretdionpolyisocyanate weisen in Abhängigkeit vom verwendeten Katalysatortyp jedoch eine mehr oder weniger hohe Eigenfarbe auf, was ihre Verwendbarkeit als Ausgangskomponente zur Herstellung hochwertiger Lackrohstoffe stark einschränkt. Es wurden daher bereits mehrfach Versuche unternommen, die Farbqualität von Uretdionpolyisocyanaten zu verbessern.

Aus der EP-A 337 116 ist bekannt, daß bei der phosphinkatalysierten Dimerisierung von aliphatischen Diisocyanaten im Ausgangsmonomeren gelöstes Kohlendioxid einen erheblichen Einfluß auf die Farbe der Produkte ausübt. Aus 1,6-Diisocyanatohexan (HDI) lassen sich beispielsweise erst nach Entfernen des gelösten Kohlendioxids (Restgehalt von weniger als 20 ppm) farbhelle Uretdionpolyisocyanate herstellen.

Die EP-A 377 177 beschreibt die Herstellung praktisch farbloser HDI-Uretdione durch Tributylphosphin-Katalyse in Gegenwart alkoholischer Co-Katalysatoren und anschließende oxidative Aufhellung der nach Dünnschichtdestillation monomerenarm anfallenden Harze mit Hilfe organischer Peroxide. Die Handhabung geeigneter Peroxide ist jedoch insbesondere im technischen Maßstab nicht unproblematisch und erfordert zum Teil erhebliche Sicherheitsvorkehrungen.

In der EP-A 569 804 wird daher vorgeschlagen zur oxidativen Aufhellung von aliphatischen oder cycloaliphatischen Uretdionpolyisocyanaten (Luft)Sauerstoff einzusetzen. Der gewünschte Effekt tritt allerdings erst oberhalb von 80 °C ein, d. h. bei Temperaturen, die bereits deutlich im Rückspaltbereich von Uretdiongruppen liegen. Aus diesem Grund lassen sich nach diesem Verfahren nur schwer monomerenarme Produkte erhalten.

Die EP-A 735 027 beschreibt eine Variante des aus der EP-A 317 744 bekannten Verfahrens zur Herstellung (cyclo)aliphatischer Uretdione durch Katalyse mit 4-Dialkylaminopyridinen, wie z.B. 4-Dimethylaminopyridin (DMAP), bei dem die Dimerisierung in Gegenwart aromatischer oder araliphatischer Phosphine oder Alkylphosphiten mit bis zu 8 Kohlenstoffatomen in den Alkylresten durchgeführt wird. Dieses modifizierte Verfahren liefert zwar Produkte verbesserter Farbqualität, diese reicht jedoch für spezielle Anwendungen, beispielsweise zur Herstellung von Uretdionpulverlackvernetzern für Polyurethan-Pulverklarlacke, häufig noch nicht aus. Darüberhinaus handelt es sich bei den in der EP-A 735 027 beschriebenen Phosphorverbindungen um relativ niedermolekulare, z.T. übel riechende und toxikologisch bedenkliche Substanzen, was deren Handhabung erschwert.

Aufgabe der vorliegenden Erfindung war es daher, ein neues Verfahren zur Herstellung von Uretdionpolyisocyanaten zur Verfügung zu stellen, das farbhelle Produkte liefert und nicht die bekannten Nachteile aufweist.

Diese Aufgabe konnte nun durch das erfindungsgemäße Verfahren gelöst werden. Das erfindungsgemäße Verfahren beruht auf der überraschenden Beobachtung, daß die Dimerisierung von aliphatischen bzw. cycloaliphatischen Diisocyanaten mit an sich bekannten Katalysatoren Uretdionpolyisocyanate mit erheblich niedrigeren Farbzahlen liefert als vergleichbare Verfahren des Standes der Technik, wenn man der Reaktionsmischung geringe Mengen spezieller trisubstituierter Phosphite zusetzt.

Gegenstand der vorliegenden Erfindung ist ein Verfahren zur Herstellung Uretdiongruppen aufweisender Polyisocyanate durch Oligomerisierung eines Teils der Isocyanatgruppen von Diisocyanaten mit aliphatisch und/oder cycloaliphatisch gebundenen Isocyanatgruppen oder Mischungen solcher Diisocyanate in Gegenwart von die Dimerisierung von Isocyanatgruppen beschleunigenden Katalysatoren- oder Katalysatorsystemen, ausgewählt aus der gruppe bestehend aus tertiären organischen Phosphinen, peralkylierten Aminophosphinen, 4-Dialkylamino-substituierten Pyridinen, Antimonpentafluorid und Bortrifluorid gegebenenfalls Abbruch der Oligomerisierungsreaktion bei einem Oligomerisierungsgrad von 10 bis 60 % durch Zusatz eines Katalysatorgiftes und gegebenenfalls Entfernung des nicht umgesetzten Diisocyanatüberschusses durch Extraktion oder Dünnschichtdestillation, dadurch gekennzeichnet, daß man die Oligomerisierungsreaktion in Gegenwart von Stabilisatoren der Formel (I) 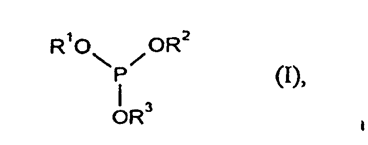 durchführt, in welcher
- R¹, R² und R³: für gleiche oder verschiedene Substituenten stehen und einen linearen oder verzweigten, gegebenenfalls Ethergruppen enthaltenden aliphatischen, einen gegebenenfalls substituierten aromatischen oder einen araliphatischen Rest mit bis zu 18 Kohlenstoffatomen bedeuten, wobei die Substituenten R¹ und R² gegebenenfalls auch gemeinsam mit dem Phosphoratom und zwei Sauerstoffatomen einen gegebenenfalls substituierten heterocyclischen 5- oder 6-Ring bilden können, wobei aber mindestens einer der Substituenten R¹, R² oder R³ für einen gegebenenfalls substituierten aromatischen Rest mit 6 bis 18 Kohlenstoffatomen oder einen linearen oder verzweigten aliphatischen Rest mit 9 bis 18 Kohlenstoffatomen steht.

Ausgangsverbindungen für das erfindungsgemäße Verfahren sind beliebige durch Phosgenierung oder nach phosgenfieien Verfahren, beispielsweise durch Urethanspaltung zugängliche Diisocyanate mit aliphatisch und/oder cycloaliphatisch gebundenen lsocyanatgruppen, insbesondere solche des Molekulargewichtsbereiches 140 bis 300, wie z.B. 1,4-Diisocyanatobutan, 1,6-Diisocyanatohexan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3-Diisocyanato-2(4)-methylcyclohexan, 1-Isocyanato-3,3,5-trimethyl-5-isocyanatomethyl-cyclohexan (Isophorondiisocyanat; IPDI), 1-Isocyanato-1-methyl-4(3)-isocyanatomethylcyclohexan, 4,4'-Diisocyanatodicyclohexylmethan sowie beliebige Gemische solcher Diisocyanate. Bevorzugt kommen HDI und/oder IPDI zum Einsatz.

Geeignete Katalysatoren oder Katalysatorsysteme für das erfindungsgemäße Verfahren sind beispielsweise tertiäre organische Phosphine (z.B. US-A 4 614 785, Kolonne 4, Zeilen 11 bis 47; DE-A 19 34 763 oder DE-A 39 00 053), peralkylierte Aminophosphine (z.B. DE-A 30 30 513, DE-A 32 27 779 oder DE-A 34 37 635) und 4-Dialkylamino-substituierte Pyridine (z.B. EP-A 317 744) aber auch Antimonpentafluorid (z.B. DE-A 34 20 114) oder Bortrifluorid (z.B. DE-A 16 70 720).

Bevorzugt kommen beim erfindungsgemäßen Verfahren als Katalysatoren oder Katalysatorsysteme die genannten tertiären organischen Phosphine oder Dialkylamino - substituierten Pyridine zum Einsatz. Ganz besonders bevorzugte Katalysatoren sind Tributylphosphin, Trioctylphosphin oder 4-Dimethylaminopyridin.

Die beim erfindungsgemäßen Verfahren verwendeten Katalysatoren werden im allgemeinen in Mengen von 0,01 bis 5 Gew.-%, vorzugsweise 0,1 bis 3 Gew.-%, bezogen auf die Menge des eingesetzten Ausgangsdiisocyanates, eingesetzt.

Gegebenenfalls können neben den genannten Katalysatoren beim erfindungsgemäßen Verfahren geeignete Cokatalysatoren mitverwendet werden (Katalysatorsysteme).

Hierbei handelt es sich beispielsweise um beliebige organische Verbindungen, die mindestens ein an Sauerstoff, Stickstoff oder Schwefel gebundenes Wasserstoffatom und einen pK_{S} von mindestens 6 aufweisen, wie sie z. B. in der DE-A 34 37 635, Seite 11, Zeile 8 bis Seite 16, Zeile 6 beschrieben sind.

Als Cokatalysatoren werden niedermolekulare, ein- oder mehrwertige Alkohole, d. h. insbesondere solche des Molekulargewichtsbereiches 32 bis 200, bzw. beliebige Gemische derartiger Alkohole bevorzugt. Geeignet sind beispielsweise Methanol, Ethanol, n-Propanol, Isopropanol, n-Butanol, n-Hexanol, 2-Ethyl-1-hexanol, 1-Methoxy-2-propanol, Ethylenglykol, Propylenglykol, die isomeren Butandiole, Hexandiole, oder Octandiole, Diethylenglykol, Dipropylenglykol, 2-Ethyl-1,3-hexandiol, 2,2,4-Trimethylpentandiol, Glycerin, Trimethylolpropan oder beliebige Gemische derartiger Alkohole.

Diese Cokatalysatoren kommen beim erfindungsgemäßen Verfahren gegebenenfalls in Mengen von bis zu 5 Gew.-%, vorzugsweise von 0,5 bis 3 Gew.-%, bezogen auf die Menge an eingesetztem Ausgangsdiisocyanat, zum Einsatz.

Die durch Reaktion der beim erfindungsgemäßen Verfahren gegebenenfalls mitzuverwendenden Cokatalysatoren mit dem Ausgangsdiisocyanat gebildeten Umsetzungsprodukte stellen die eigentlichen Cokatalysatoren dar. Hieraus folgt, daß prinzipiell anstelle der beispielhaft genannten Cokatalysatoren auch deren separat hergestellte Umsetzungsprodukte mit Isocyanaten, beispielsweise durch Umsetzung der bevorzugt eingesetzten alkoholischen Cokatalysatoren mit Ausgangsdiisocyanat erhaltene Urethane, als Cokatalysatoren geeignet sind.

Erfindungsgemäß wird die Oligomerisierungsreaktion beim erfindungsgemäßen Verfahren in Gegenwart spezieller Phosphitstabilisatoren durchgeführt. Hierbei handelt es sich um an sich bekannte trisubstituierte Phosphitstrukturen der Formel (I) aufweisende Verbindungen. 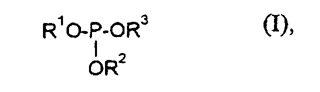 in welcher
R¹, R² und R³ für gleiche oder verschiedene Substituenten stehen und einen linearen oder verzweigten, gegebenenfalls Ethergruppen enthaltenden aliphatischen, einen gegebenenfalls substituierten aromatischen oder einen araliphatischen Rest mit bis zu 18 Kohlenstoffatomen bedeuten, wobei die Substituenten R¹ und R² gegebenenfalls auch gemeinsam mit dem Phosphoratom und zwei Sauerstoffatomen einen gegebenenfalls substituierten heterocyclischen 5- oder 6-Ring bilden können, wobei aber mindestens einer der Substituenten R¹, R² oder R³ für einen gegebenenfalls substituierten aromatischen Rest mit 6 bis 18 Kohlenstoffatomen oder einen linearen oder verzweigten aliphatischen Rest mit 9 bis 18 Kohlenstoffatomen steht.

Geeignete Stabilisatoren sind beispielsweise Arylphosphite, wie z.B. Triphenylphosphit oder Tris(nonylphenyl)phosphit, Alkyl-arylphosphite, wie z.B. Diphenyl-isooctylphosphit, Diphenyl-isodecylphosphit, Diisodecyl-phenylphosphit, Diisooctyloctylphenylphosphit, Phenyl-neopentylglykolphosphit oder 2,4,6-Tri-tert.-butylphenyl-(2-butyl-2-ethyl-1,3-propandiol)phosphit, Alkylphosphite, wie z.B. Triisodecylphosphit, Trilaurylphosphit oder Tris(tridecyl)phosphit, oder aromatisch bzw. aliphatisch substituierte Diphosphite, wie z. B. Diisodecyl-pentaerythritdiphosphit, Distearyl-pentaerythritdiphosphit, Bis(2,4-di-tert.-butylphenyl)-pentaerythritdiphosphit oder Tetraphenyl-dipropylenglykoldiphosphit.

Bevorzugte Phosphitstabilisatoren sind solche, bei denen mindestens einer der Substituenten R¹, R² oder R³ ein linearer oder verzweigter aliphatischer Rest mit 10 bis 16 Kohlenstoffatomen oder ein Phenylrest ist. Besonders bevorzugt sind Triisodecylphosphit, Phenyldiisodecylphospit und/oder Diphenylisodecylphospit.

Diese Stabilsatoren der Formel (I) werden beim erfindungsgemäßen Verfahren in Mengen von 0,01 bis 10 Gew.-%, vorzugsweise von 0,1 bis 5 Gew.-% und besonders bevorzugt von 0,5 bis 3 Gew.-%, bezogen auf die Menge an eingesetztem Ausgangsdiisocyanat, eingesetzt.

Gegebenenfalls wird die Oligomerisierungsreaktion beim erfindungsgemäßen Verfahren mit Hilfe geeigneter Katalysatorgifte abgebrochen, wenn 10 bis 60 % (Umsetzungsgrad) der ursprünglich im Ausgangsgemisch vorliegenden Isocyanatgruppen abreagiert haben. Solche Katalysatorgifte sind beispielsweise Alkylierungsmittel wie Dimethylsulfat oder p-Toluolsulfonsäuremethylester, Acylierungsmittel wie Benzoylchlorid, Säuren wie Perfluorbutansulfonsäure, Schwefel oder Sulfonylisocyanate (z.B. US-PS 4 614 785, Kolonne 5, Zeile 27 bis Kolonne 6, Zeile 35), silylierte Säuren (z.B. EP-A 520 210).

Die zur Abstoppung der Reaktion benötigte Menge des Katalysatorgiftes richtet sich dabei nach der Menge des verwendeten Katalysators; im allgemeinen wird eine äquimolare Menge des Abstoppers, bezogen auf den zu Beginn eingesetzten Dimerisierungskatalysator, eingesetzt. Berücksichtigt man allerdings eventuell während der Reaktion auftretende Katalysatorverluste, so können zur Reaktionsstoppung auch schon 20 bis 80 Äquivalent-% des Katalysatorgiftes, bezogen auf die ursprünglich eingesetzte Katalysatormenge, ausreichen.

Das erfindungsgemäße Verfahren wird vorzugsweise in Substanz durchgeführt. Es kann aber auch in Gegenwart von gegenüber Isocyanatgruppen inerten Lösungsmitteln durchgeführt werden. Geeignete Lösungsmittel sind beispielsweise Hexan, Toluol, Xylol, Chlorbenzol, Essigsäureethylester, Essigsäurebutylester, Ethylglykolacetat, Propylenglykolmonomethyletheracetat, Aceton, Methylisobutylketon, Methylenchlorid, N-Methylpyrrolidon oder beliebige Gemische derartiger Lösungsmittel.

Zur Durchführung des erfindungsgemäßen Verfahrens wird das Ausgangsdiisocyanat bzw. eine Mischung verschiedener Ausgangsdiisocyanate gemeinsam mit einem Phosphitstabilisator der genannten Art oder einer Mischung solcher Stabilisatoren, gegebenenfalls unter Inertgas wie beispielsweise Stickstoff und gegebenenfalls in Gegenwart eines geeigneten Lösungsmittels der genannten Art auf eine Temperatur zwischen 20 und 100°C, vorzugsweise 20 bis 70°C erwärmt. Dann kann der gegebenenfalls mitzuverwendende, bevorzugt alkoholische Cokatalysator zugemischt werden. Im Anschluß hieran wird, gegebenenfalls nach Beendigung der spontan ablaufenden Reaktion zwischen Cokatalysator und Ausgangsdiisocyanat, ein Dimerisierungskatalysator der beispielhaft genannten Art in der oben angegebenen Menge zugegeben und die Reaktionstemperatur gegebenenfalls durch eine geeignete Maßnahme (Heizen oder Kühlen) auf eine Temperatur von 20 bis 120°C, vorzugsweise von 25 bis 80°C eingestellt. Die Umsetzung kann gegebenenfalls bei Erreichen eines Oligomerisierungsgrades von 10 bis 60 %, vorzugsweise 10 bis 40 % durch Zugabe eines Katalysatorgiftes der beispielhaft genannten Art und gegebenfalls nachfolgendes kurzzeitiges Erhitzen der Reaktionsmischung auf eine oberhalb 80°C, vorzugsweise oberhalb 120°C liegende Temperaturen beendet werden.

Unter "Oligomerisierungsgrad" ist dabei der Prozentsatz der in der Ausgangsmischung ursprünglich vorhandenen Isocyanatgruppen zu verstehen, der während der erfindungsgemäßen Umsetzung (insbesondere durch Dimerisierung, daneben unter Trimerisierung und im Falle der Mitverwendung der beschriebenen, beispielsweise alkoholischen Cokatalysatoren durch Reaktion mit Isocyanatgruppen, beispielsweise unter Urethanisierung) verbraucht wird. Der genannte Oligomerisierungsgrad wird im allgemeinen nach einer Reaktionszeit von 1 bis 48, vorzugsweise 2 bis 24 Stunden erreicht.

Vorzugsweise wird das Reaktionsgemisch anschließend durch Dünnschichtdestillation im Hochvakuum (z.B. <1 mbar) unter möglichst schonenden Bedingungen, beispielsweise bei einer Temperatur von 100 bis 200°C, vorzugsweise von 120 bis 180°C, von flüchtigen Bestandteilen (überschüssigen monomeren Diisocyanaten, gegebenenfalls mitverwendeten Lösungsmitteln, gegebenenfalls den eingesetzten Phosphitstabilisatoren und, bei Verzicht auf den Einsatz eines Katalysatorgiftes, gegebenenfalls aktivem Katalysator) befreit.

Weiterhin können die genannten flüchtigen Bestandteile durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Oligomerisierungsprodukt abgetrennt werden.

Auf diese Weise erhält man in Abhängigkeit von der Art des eingesetzten Ausgangsdiisocyanates bei Raumtemperatur flüssige oder hochviskose Uretdiongruppen aufweisende Polyisocyanatgemische mit einem Gehalt an aliphatisch und/oder cycloaliphatisch gebundenen Isocyanatgruppen von 10 bis 30 Gew.-%, vorzugsweise von 15 bis 25 Gew.-%, die im allgemeinen weniger als 5 Gew.-%, vorzugsweise weniger als 2 Gew.-%, besonders bevorzugt weniger als 1 Gew.-% an monomeren Ausgangsdiisocyanaten enthalten. Diese Produkte sind im Vergleich zu Uretdionpolyisocyanaten, die nach entsprechenden Dimerisierungsverfahren des Standes der Technik ohne den Zusatz der erfindungswesentlichen Phosphitstabilisatoren hergestellt wurden wesentlich farbheller (erheblich geringere Eigenfarbe).

Die anfallenden Destillate, die neben den nicht umgesetzten monomeren Ausgangsdiisocyanaten und gegebenenfalls mitverwendeten Lösungsmitteln gegebenenfalls Phosphitstabilisator sowie, bei Verzicht auf den Einsatz eines Katalysatorgiftes, gegebenenfalls aktiven Katalysator enthalten, können problemlos zur erneuten Oligomerisierung verwendet werden.

Gegebenfalls kann beim erfindungsgemäßen Verfahren nach anteiliger katalytischer Dimerisierung und Abbruch der Reaktion beim angestrebten Oligomerisierungsgrad durch Zugabe eines Katalysatorgiftes auch auf die Abtrennung der überschüssigen, nicht umgesetzten Diisocyanate verzichtet werden. In diesem Fall erhält man als Verfahrensprodukte klare, farbhelle Lösungen von Uretdiongruppen aufweisenden Polyisocyanaten in bis zu 70 Gew.-% monomerem Ausgangsdiisocyanat.

Die nach dem erfindungsgemäßen Verfahren erhältlichen Uretdionpolyisocyanate bzw. deren Lösungen in monomeren Ausgangsdiisocyanaten stellen wertvolle Ausgangsmaterialien zur Herstellung von Polyurethankunststoffen nach dem Polyadditionsverfahren, bevorzugt zur Herstellung von Ein- oder ZweikomponentenPolyurethanlacken, dar. Sie eignen sich insbesondere als Isocyanatkomponente zur Herstellung von Uretdionpulverlackvernetzern.

### Beispiele

Alle Prozentangaben beziehen sich, soweit nichts anderslautendes vermerkt, auf das Gewicht.

Die Bestimmung der HAZEN-Farbzahlen erfolgte mit Hilfe eines Neo-Komparators der Fa. Hellige (Freiburg).

### Beispiel 1

1000 g (5,95 mol) Hexamethylendiisocyanat (HDI) werden bei Raumtemperatur unter trockenem Stickstoff nacheinander mit 5 g (0,5 %) Diisodecylphenylphosphit als Stabilisator, 10 g (1,0 %) 1,3-Butandiol als Co-Katalysator sowie 3 g (0,3 % / 0,015 mol) Tri-n-butylphosphin als Katalysator versetzt und anschließend auf 60°C erwärmt. Nach einer Reaktionszeit von 4,5 Stunden beträgt der NCO-Gehalt des Reaktionsgemisches 42,0 %, entsprechend einem Oligomerisierungsgrad von 14,5 %. Die Reaktion wird durch Zugabe von 2,8 g (0,015 mol) p-Toluolsulfonsäuremethylester und einstündiges Erhitzen auf 80°C abgestoppt. Nach Dünnschichtdestillation bei einer Temperatur von 140°C und einem Druck von 0,5 mbar erhält man ein farbloses uretdiongruppenhaltiges Polyisocyanat mit einem NCO-Gehalt von 21,5 %, einem Gehalt an monomerem HDI von 0,3 %, einer Viskosität (nach DIN 53 018) von 240 mPas (23°C) und einer HAZEN-Farbzahl von 15 - 20.

Zum Vergleich wird auf analoge Weise jedoch ohne Mitverwendung von Diisodecylphenylphosphit ein HDI-Polyisocyanat hergestellt. Das schwach gelbe Harz weist einen NCO-Gehalt von 21,6 %, einen Gehalt an monomerem HDI von 0,2 %, eine Viskosität (nach DIN 53 018) von 240 mPas (23°C) und eine HAZEN-Farbzahl von 50 auf. Der Vergleich zeigt, daß die erfindungsgemäße Dimerisierung in Gegenwart des Phosphit-Stabilisators ein deutlich helleres Produkt liefert.

### Beispiel 2

1000 g (4,50 mol) Isophorondiisocyanat (IPDI) werden bei Raumtemperatur unter trockenem Stickstoff und Rühren nacheinander mit 10 g (1 %) Triphenylphosphit als Stabilisator sowie 20 g (2 %) 4-Dimethylaminopyridin (DMAP) als Katalysator versetzt. Nach 20 h wird die hellgelbe Reaktionsmischung, die einen NCO-Gehalt von 28,7 %, entsprechend einem Oligomerisierungsgrad von 21,8 % aufweist, ohne vorherige Zugabe eines Katalysatorgiftes mit Hilfe eines Dünnschichtverdampfers bei einer Temperatur von 160°C und einem Druck von 0,3 mbar von flüchtigen Bestandteilen befreit. Man erhält ein gelbes Uretdionpolyisocyanat mit einem Gehalt an freien NCO-Gruppen von 17,8 %, einem Gehalt an monomerem IPDI von 0,3 %, einer Viskosität (nach DIN 53 018) von mehr als 200000 mPas (23°C) und einer HAZEN-Farbzahl, bestimmt an einer 10 %-igen Lösung in Methylenchlorid, von 30.

Ein zum Vergleich ohne Mitverwendung des Stabilisators gemäß EP-A 317 744 hergestelltes, ebenfalls hochviskoses gelbes IPDI-Uretdion mit einem NCO-Gehalt von 17,5 % und einem Gehalt an monomerem HDI von 0,3 % weist als 10 %-ige Lösung in Methylenchlorid eine HAZEN-Farbzahl von 70 auf Der Vergleich zeigt, daß die erfindungsgemäße Dimerisierung in Gegenwart des Phosphit-Stabilisators ein erheblich helleres Produkt liefert.

### Beispiele 3 - 8

Nach dem in Beispiel 2 beschriebenen Verfahren wurden unter Verwendung unterschiedlicher Phosphit-Stabilisatoren IPDI-Uretdione hergestellt. Der Oligomerisierungsgrad betrug in allen Fällen zwischen 21 und 22 %. Tabelle 1 zeigt die eingesetzten Mengen an Katalysator und Stabilisator (jeweils bezogen auf die Menge an eingesetztem Ausgangsdiisocyanat) sowie die Kenndaten der nach Dünnschichtdestillation erhaltenen hochviskosen Harze.

Die Vergleichsbeispiele 7 und 8 zeigen, daß die erfindungsgemäß hergestellten Uretdionpolyisocyanate gegenüber solchen, die unter Verwendung der in der EP-A 735 027 beschriebenen dreiwertigen Phosphorverbindungen hergestellt wurden, eine nochmals deutlich verbesserte Farbqualität aufweisen.

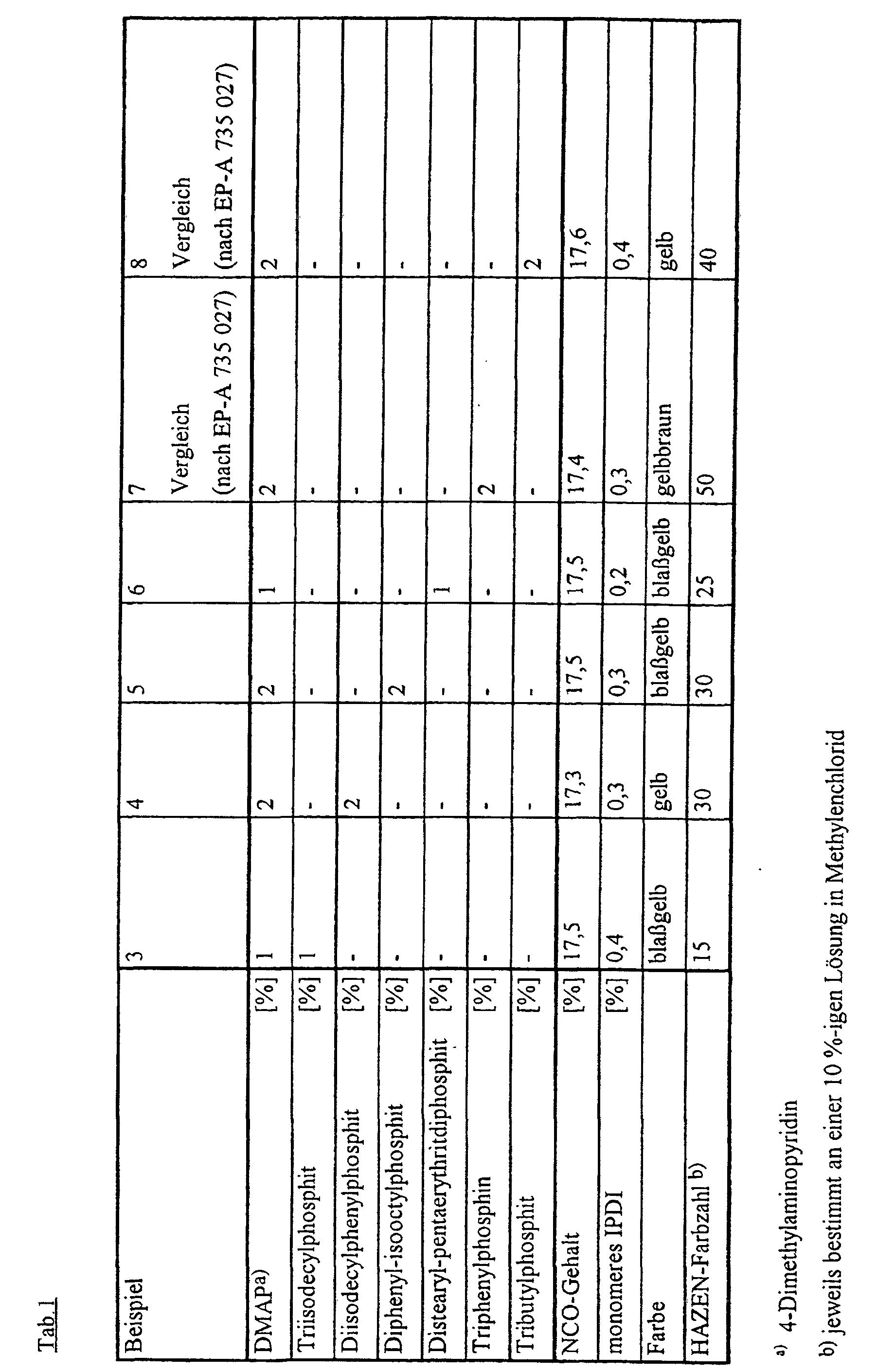

## Patentansprüche

1. Verfahren zur Herstellung Uretdiongruppen aufweisender Polyisocyanate durch Oligomerisierung eines Teils der Isocyanatgruppen von Diisocyanaten mit aliphatisch und/oder cycloaliphatisch gebundenen Isocyanatgruppen oder Mischungen solcher Diisocyanate in Gegenwart von die Dimerisierung von Isocyanatgruppen beschleunigenden Katalysatoren ausgewählt aus der gruppe bestehend aus tertiären organischen Phosphinen, peralkylierten Aminophosphinen, 4-Dialkylamino-substituierten Pyridinen, Antimonpentafluorid und Bortrifluorid oder Katalysatorsystemen, gegebenenfalls Abbruch der Oligomerisierungsreaktion bei einem Oligomerisierungsgrad von 10 bis 60 % durch Zusatz eines Katalysatorgiftes und gegebenenfalls Entfernung des nicht umgesetzten Diisocyanatüberschusses durch Extraktion oder Dünnschichtdestillation, **dadurch gekennzeichnet, daß** man die Oligomerisierungsreaktion in Gegenwart von Stabilisatoren der Formel (I) 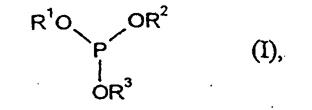 durchführt, in welcher
R¹, R² und R³ für gleiche oder veschiedene Substituenten stehen und einen linearen oder verzweigten, gegebenenfalls Ethergruppen enthaltenden aliphatischen, einen gegebenenfalls substituierten aromatischen oder einen araliphatischen Rest mit bis zu 18 Kohlenstoffatomen bedeuten, wobei die Substituenten R¹ und R² gegebenenfalls auch gemeinsam mit dem Phosphoratom und zwei Sauerstoffatomen einen gegebenenfalls substituierten heterocyclischen 5- oder 6-Ring bilden können, wobei aber mindestens einer der Substituenten R¹, R² oder R³ für einen gegebenenfalls substituierten aroamtischen Rest mit 6 bis 18 Kohlenstoffatomen oder einen linearen oder verzweigten aliphatischen Rest mit 9 bis 18 Kohlenstoffatomen steht.

2. Verfahren gemäß Anspruch 1, **dadurch gekennzeichnet, daß** man als Oligomerisierungskatalysator Trialkylphosphine und/oder 4-Dialkylaminopyridine verwendet.

3. Verfahren gemäß Anspruch 1, **dadurch gekennzeichnet, daß** man als Oligomerisierungskatalysator Tributylphosphin oder Trioctylphosphin verwendet.

4. Verfahren gemäß Anspruch 1, **dadurch gekennzeichnet, daß** man als Oligomerisierungskatalysator 4-Dimethylaminopyridin verwendet.

5. Verfahren gemäß Anspruch 1, **dadurch gekennzeichnet, daß** man als Diisocyanate 1,6-Diisocyanatohexan und/oder 1-Isocyanato-3,3,5-trimethyl-5-isocyanatomethyl-cyclohexan verwendet.

6. Verfahren gemäß Anspruch 1, **dadurch gekennzeichnet, daß** man als Stabilsator Triisodecylphosphit, Phenyldiisodecylphospit und/oder Diphenylisodecylphospit verwendet.

## Claims

1. Process for preparing polyisocyanates containing uretdione groups by oligomerizing some of the isocyanate groups of diisocyanates with aliphatically and/or cycloaliphatically attached isocyanate groups or mixtures of such diisocyanates in the presence of catalysts which accelerate the dimerization of isocyanate groups and are selected from the group consisting of tertiary organic phosphines, peralkylated aminophosphines, 4-dialkylamino-substituted pyridines, antimony pentafluoride and boron trifluoride, or catalyst systems, terminating the oligomerization reaction if desired at a degree of oligomerization of 10% to 60% by adding a catalyst poison and removing, where appropriate, the unreacted diisocyanate excess by extraction or thin-film distillation, **characterized in that** the oligomerization reaction is carried out in the presence of stabilizers of the formula (I) 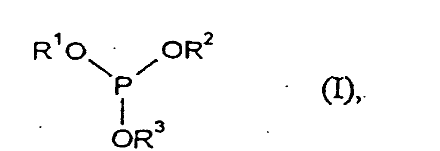 in which
R¹, R² and R³ are identical or different substituents and are a linear or branched aliphatic radical optionally containing ether groups, or are an optionally substituted aromatic radical or an araliphatic radical having up to 18 carbon atoms, it being possible for the substituents R¹ and R², where appropriate also together with the phosphorus atom and two oxygen atoms, to form an optionally substituted heterocyclic 5- or 6-membered ring, but at least one of the substituents, R¹, R² or R³, is an optionally substituted aromatic radical having 6 to 18 carbon atoms or a linear or branched aliphatic radical having 9 to 18 carbon atoms.

2. Process according to Claim 1, **characterized in that** trialkylphosphines and/or 4-dialkylaminopyridines are used as oligomerization catalyst.

3. Process according to Claim 1, **characterized in that** tributylphosphine or trioctylphosphine is used as oligomerization catalyst.

4. Process according to Claim 1, **characterized in that** 4-dimethylaminopyridine is used as oligomerization catalyst.

5. Process according to Claim 1, **characterized in that** 1,6-diisocyanatohexane and/or 1-isocyanato-3,3,5-trimethyl-5-isocyanatomethylcyclohexane are/is used as diisocyanate(s).

6. Process according to Claim 1, **characterized in that** triisodecyl phosphite, phenyl diisodecyl phosphite and/or diphenyl isodecyl phosphite are/is used as stabilizer.

## Revendications

1. Procédé pour la préparation de polyisocyanates présentant des groupes uretdion par oligomérisation d'une partie des groupes isocyanates de diisocyanates avec des groupes isocyanates aliphatiquement et/ou cycloaliphatiquement liés ou des mélanges de tels diisocyanates en présence de catalyseurs ou de systèmes de catalyseurs accélérant la dimérisation des groupes isocyanates choisis parmi des phosphines organiques tertiaires, des aminophosphines peralkylées, des pyridines 4-dialkylamino-substituées, le pentafluorure d'antimoine et le trifluorure de bore, éventuellement par interruption de la réaction d'oligomérisation à un degré d'oligomérisation compris entre 10 et 60 % par addition d'un poison de catalyseur et éventuellement par élimination de l'excès de diisocyanate n'ayant pas réagi par extraction ou distillation en couche mince, **caractérisé en ce que** l'on réalise la réaction d'oligomérisation en présence de stabilisateurs de la formule (I) 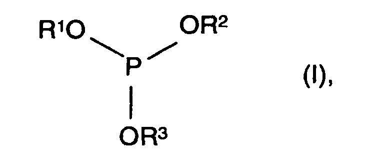 dans laquelle
R¹, R² et R³ représentent des substituants identiques ou différents et un reste linéaire ou ramifié, aliphatique contenant éventuellement des groupes éther, un reste aromatique éventuellement substitué ou un reste araliphatique avec jusqu'à 18 atomes de carbone, les substituants R¹ et R² pouvant éventuellement également former en association avec l'atome de phosphore et deux atomes d'oxygène un noyau 5- ou 6- hétérocyclique éventuellement substitué, au moins un des substituants R¹, R² ou R³ représentant toutefois un reste aromatique éventuellement substitué avec de 6 à 18 atomes de carbone ou un reste aliphatique linéaire ou ramifié avec de 9 à 18 atomes de carbone.

2. Procédé selon la revendication 1, **caractérisé en ce que** l'on utilise comme catalyseur d'oligomérisation des trialkylphosphines et/ou des 4-dialkylaminopyridines.

3. Procédé selon la revendication 1, **caractérisé en ce que** l'on utilise comme catalyseur d'oligomérisation la tributylphosphine ou la trioctylphosphine.

4. Procédé selon la revendication 1, **caractérisé en ce que** l'on utilise comme catalyseur d'oligomérisation la 4-diméthylaminopyridine.

5. Procédé selon la revendication 1, **caractérisé en ce que** l'on utilise comme diisocyanate le 1,6-diisocyanatohexane et/ou le 1-isocyanato-3,3,5-triméthyl-5-isocyanatométhyl-cyclohexane.

6. Procédé selon la revendication 1, **caractérisé en ce que** l'on utilise comme stabilisateur le phosphite de triisodécyle, le phosphite de phényldiisodécyle et/ou le phosphite de diphénylisodécyle.
